# EUROPÄISCHE PATENTANMELDUNG

(11) **EP 2 402 056 A1**
(43) Veröffentlichungstag der Anmeldung: **04.01.2012**
(21) Anmeldenummer: 11164523.0
(22) Anmeldetag: 03.05.2011
(51) Int. Cl.: A61N 5/00

(54) **Generatoranordnung zur Erzeugung von Energiewellen**

(30) Priorität: 29.06.2010 DE 202010007933 U
(71) Anmelder: Kreft, Siegfried, 99189 Elxleben (DE); Helmis, Gabriele, 99195 Stotternheim (DE)
(72) Erfinder: Kreft, Siegfried, 99189 Elxleben (DE); Helmis, Gabriele, 99195 Stotternheim (DE)
(74) Vertreter: Engel, Christoph Klaus

(57) **Zusammenfassung**

Die Erfindung betrifft eine Generatoranordnung zur Erzeugung von Energiewellen, umfassend eine Oszillatorschaltung mit einem ersten und einem zweiten Anregungsgenerator (03), welche jeweils hochfrequente Anregungswellen über je eine vorgeschaltete Spule (05) an je eine Antenne (07) abgeben. Die Generatoranordnung zeichnet sich dadurch aus, dass die beiden Antennen (07) elektromagnetisch an einen Resonanzkörper (01) gekoppelt sind, in welchem sich in Reaktion auf die eingeprägten Anregungswellen Resonanzwellen ausbilden.

## Beschreibung

Die Erfindung betrifft eine Generatoranordnung zur Erzeugung von Energiewellen gemäß dem Oberbegriff des Anspruchs 1.

Auf der Grundlage der Entdeckung eines fraktalen Musters in den Eigenschwingungsfrequenzen der Hauptmassenträger, der Protonen, wurden Erkenntnisse zu den sogenannten Scaling Phänomenen gewonnen, die in allen Bereichen der Naturwissenschaften von Bedeutung sind. Scaling Phänomene haben ihre Ursache in Eigenresonanzschwingungen. Solche Eigenresonanzschwingungen wiederum haben logarithmisch fraktale Frequenzspektren und entwickeln sich auf energetisch niedrigstem Niveau. Der Grund für die Vielzahl der Scaling Phänomene liegt in einem auf energetisch niedrigstem Niveau existierenden Kompressionswellensystem, dessen Spektrum logarithmisch und fraktal ist. Diese Kompressionswellen determinieren alle Vorgänge in der Natur und sind zudem Ursache für jegliche Materieakkumulation im Raum.

Die physikalischen Parameter analysierter Systeme der Natur weisen Werte auf, die besonderen Qualitäten des fundamentalen Fraktals entsprechen. Die Global Scaling Analyse ist in der Lage, wichtige Kriterien für die Diagnose des Gesundheitszustandes eines Mensches zu liefern. Gleichzeitig ist es möglich, durch geeignete Maßnahmen das Frequenzspektrum physiologischer oder zellbiologischer Prozesse zu korrigieren und so therapeutische Effekte zu erzielen.

Zellbiologische Studien des Instituts für Theoretische und Experimentelle Biophysik am Pushchino Scientific Center der Russischen Akademie der Wissenschaften haben gezeigt, dass die Protonenresonanz-Modulationsfrequenz von 101 Hz die Aktivität der Sukzinatdehydrogenase erhöht, die ihrerseits Bernsteinsäure in den Mitochondrien verbrennt. Die Verbrennung von Bernsteinsäure in den Mitochondrien ist die mächtigste Energiequelle der Zelle. Basierend auf diesen Erkenntnissen ist ein Therapiesystem, das ProtoLight Therapie System, durch das Institut für Raum-Energie-Forschung GmbH, München, Deutschland in Zusammenarbeit mit dem Hersteller ROM-Elektronik entwickelt worden. Das Therapiesystem generiert monochromatisches rotes bzw. infrarotes Licht in der Nähe der Protonensubresonanz-Wellenlänge 754 nm, welche mit zellbiologisch relevanten Protonenresonanzfrequenzen moduliert ist. Das auf diese Weise durch das Therapiesystem generierte Licht bringt Protonenresonanzen in das Zellgewebe, welche gezielt gesundheitsfördernde biochemische Prozesse stimulieren oder andere Prozesse hemmen. Das Therapiesystem nutzt die Modulationsfrequenz 101 Hz zur Stimulation der mitochondrialen Energieproduktion, zur Beschleunigung der Wundheilung und der Zellregeneration. Das System wird auch in der Veterinärmedizin erfolgreich angewendet, da das protonenresonanzmodulierte Licht auf zellbiologischer Ebene wirkt.

Die Erkenntnisse der Global Scaling Theorie lassen sich aber auch auf andere Prozesse zur Energiewandlung und -gewinnung anwenden.

Die Aufgabe der Erfindung besteht darin, eine Generatoranordnung zur Erzeugung von Energiewellen mit speziellen Welleneigenschaften bereitzustellen, insbesondere zur Erzeugung sogenannter Scalarwellen. Eine Teilaufgabe der Erfindung besteht darin, eine optimierte Generatoranordnung zu schaffen, welche Energiewellen abstrahlt, die auf den Organismus eine gesundheitsfördernde und/oder therapeutische Wirkung entfalten. Insbesondere sollen diese Energiewellen auf Zellenniveau durch Ausgleich des Energiehaushaltes positiv wirken.

Die genannte Aufgabe wird durch eine Generatoranordnung zur Erzeugung von Energiewellen gemäß dem Anspruch 1 gelöst.

Die erfindungsgemäße Generatoranordnung umfasst eine Oszillatorschaltung mit einem ersten und einem zweiten Anregungsgenerator, welche jeweils hochfrequente Anregungswellen über je eine vorgeschaltete Spule an je eine Antenne abgeben. Die beiden Antennen sind elektromagnetisch an einen Resonanzkörper gekoppelt, in welchem sich in Reaktion auf die eingeprägten Anregungswellen Resonanzwellen als die gewünschten Energiewellen ausbilden. Zur Entstehung der Resonanzwellen kommt es dadurch, dass die Anregungswellen den Resonanzkörper zum Schwingen bringen und infolge dessen Resonanzwellen entstehen, welche sich um den Resonanzkörper herum ausbreiten.

Die den Antennen vorgeschalteten Spulen können als bifilar gewickelte Spulen ausgeführt werden, wobei die Antennen vorzugsweise als Dipol ausgebildet sind. Bei den von den Dipolen abgestrahlten Anregungswellen kann es sich um transversale und longitudinale Wellen handeln.

Vorzugsweise besteht der Resonanzkörper aus einem gesinterten Materialgemisch aus Kunststoff und Quarzsand, insbesondere aus einem Material, welches gemäß dem Europäischen Patent EP 1 299 321 hergestellt wurde. In Folge der Anregungswellen wird der Resonanzkörper zum Schwingen und Abstrahlen von Resonanzwellen gezwungen. Die Resonanzwellen verbreiten sich in die Umgebung des Resonanzkörpers und können insbesondere therapeutische Wirkung auf Personen entfalten, die im direkten körperlichen Kontakt mit dem Resonanzkörper sind oder sich in dessen Umgebung aufhalten. Insbesondere beträgt der Wirkungsbereich bis zu 2,7 m im Umkreis des Standortes des Resonanzkörpers. Die abgestrahlten Resonanzwellen haben positiven Einfluss auf den Energiehaushalt der Zellen und damit des ganzen Organismus der zu behandelnden Personen. Dadurch lässt sich eine heilende Wirkung bei Kranken mit unterschiedlichen Beschwerden feststellen.

In einer bevorzugten Ausführungsform der erfindungsgemäßen Generatoranordnung ist am Resonanzkörper eine Messspule angebracht, wobei die darin induzierte Spannung ein Maß für die therapeutische Wirkung der Resonanzwellen auf die behandelte Person ist. Die Messspule kann außen um den Resonanzkörper gewickelt angeordnet werden, wobei die von der Messspule abgegebene Spannung vorzugsweise über eine Graetzbrücke gleichgerichtet wird.

Weitere Einzelheiten und Vorteile ergeben sich aus der nachfolgenden Beschreibung einer bevorzugten Ausführungsform der erfindungsgemäßen Generatoranordnung, unter Bezugnahme auf die Zeichnung. Es zeigen:
- Fig. 1: ein Blochschaltbild einer Generatoranordnung zur Erzeugung von Energiewellen;
- Fig. 2: eine schematische Darstellung der Generatoranordnung.

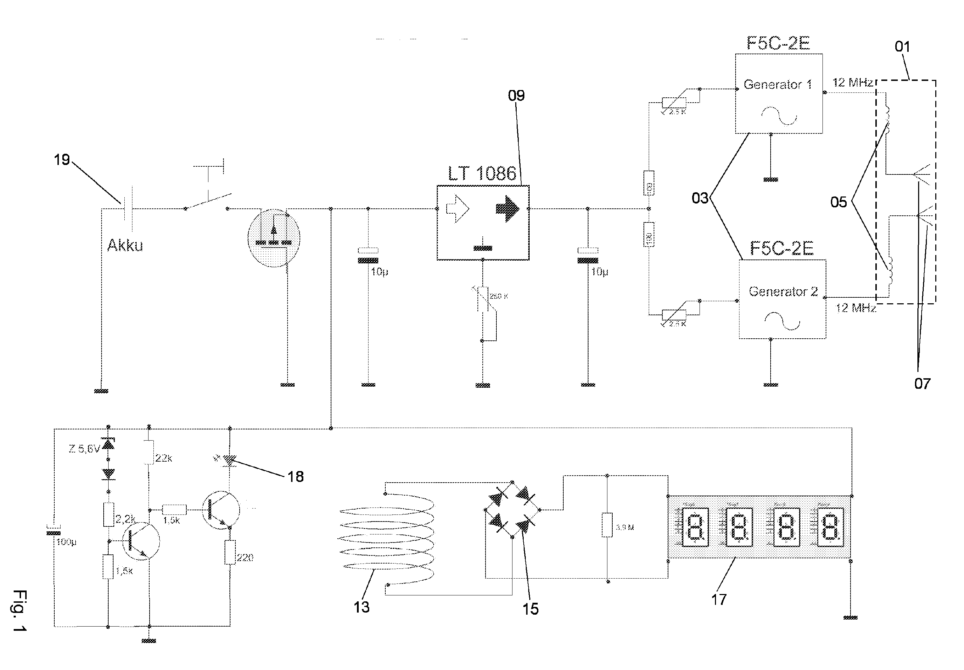

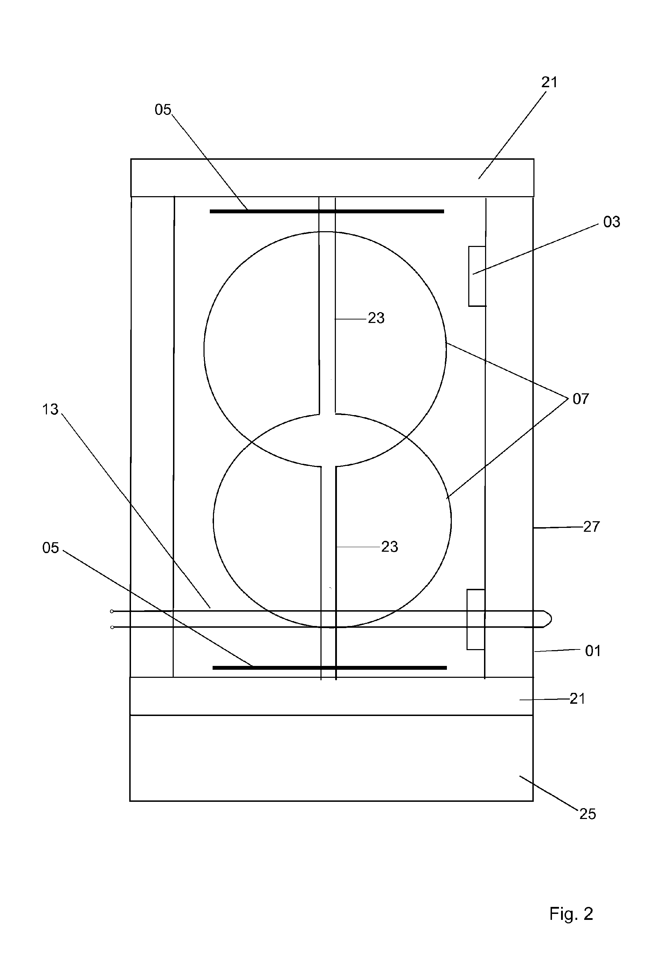

In Fig. 1 ist eine Generatoranordnung dargestellt, welche mit einem Resonanzkörper 01 zusammenwirkt. Die Positionierung des Resonanzkörpers relativ zu weiteren Bestandteilen der Generatoranordnung wird weiter unten in Bezug auf die Fig. 2 näher beschrieben. Der Resonanzkörper 01 besteht aus einem Material, welches gemäß dem Europäischen Patent EP 1 299 321 hergestellt wurde. Die Zusammensetzung und die Herstellungsweise des Resonanzkörpers sind aus dieser Europäischen Patentschrift zu entnehmen, die insoweit in die Offenbarung einbezogen wird. Der Werkstoff, aus welchem der Resonanzkörper hergestellt ist, besteht insbesondere aus Sand und Polymeren und gegebenenfalls weiteren Zuschlagstoffen. Dieser Werkstoff wird vorzugsweise folgendermaßen hergestellt:
- Erhitzen des Sandes auf eine Temperatur zwischen 300°C und 800°C;
- Zuführen des erhitzten Sandes und des Polymers in einen geschlossenen Mischkessel, der eine Erdungselektrode und ein Überdruckventil besitzt, unter Aufrechterhaltung einer Mischtemperatur, die oberhalb der Schmelztemperatur des Polymers liegt;
- Mischen des Sandes und des Polymers zur Erzeugung eines heißen Schmelzgemisches;
- Einbringen des Schmelzgemisches in eine Pressform;
- Abkühlen des Schmelzgemisches unter Druck bis zum Erstarren des Schmelzgemisches in der Pressform;
- weiteres Abkühlen des erstarrten Werkstoffes unter Normaldruck.

Die Generatoranordnung weist eine Oszillatorschaltung mit einem ersten und einem zweiten Anregungsgenerator 03 auf. Diese geben hochfrequente Anregungswellen über je eine Spule 05 an je eine Antenne 07 ab. Die Ansteuerung der Anregungsgeneratoren 03 erfolgt über einen Oszillatorschaltkreis 09, wobei die abgegebenen Anregungswellen eine Frequenz von 10 bis 15 MHz, insbesondere 12 MHz besitzen. Die Spulen 05 sind hier vorzugsweise als bifilar gewickelte Spulen ausgebildet, wobei diese mit jeweils 5 bis 15 Windungen, insbesondere 9 Windungen gewickelt sind. Die Antennen 07 sind als Dipole ausgebildet. Bei den von den Dipolen abgestrahlten Anregungswellen handelt es sich um transversale und longitudinale Wellen.

Die durch die Antennen 07 abgestrahlten Anregungswellen bringen den Resonanzkörper 01 zum Schwingen, wodurch im Resonanzkörper 01 Energiewellen entstehen, die als Resonanzwellen abgestrahlt werden. Damit ist der Resonanzkörper 01 gleichzeitig ein Strahler von Resonanzwellen. Es wurde festgestellt, dass die abgestrahlten Resonanzwellen einen positiven therapeutischen Einfluss auf Personen haben, welche zur Behandlung in direktem Kontakt mit dem Resonanzkörper 01 stehen oder sich in dessen Umgebung aufhalten.

Am Resonanzkörper 01 ist weiterhin eine Messspule 13 angebracht, wobei die darin induzierte Spannung ein Maß für die abgestrahlten Resonanzwellen und damit auch für die therapeutische Wirkung der Resonanzwellen auf die zu behandelnde Person ist. Die Messspule 13 ist z.B. außen um den Resonanzkörper 01 gewickelt angeordnet, wobei die von der Messspule 13 abgegebene Spannung über eine Graetzbrücke 15 gleichgerichtet und deren Betrag an einer Anzeigeeinheit, z.B. einem digitalen Display 17 angezeigt wird. Außerdem ist eine Betriebsanzeige 18 mit zugehörigen Schaltungselementen vorgesehen, welche die Inbetriebnahme der Generatorschaltung signalisiert und die Versorgungsspannung überwacht.

Die Stromversorgung der Generatoranordnung und ggf. auch der Anzeigeeinheit erfolgt beispielsweise über einen Kleinakkumulator 19 oder eine vergleichbare Stromversorgung (vorzugsweise 9 V/150 mA).

Fig. 2 zeigt in einer vereinfachten Schnittdarstellung die erfindungsgemäße Generatoranordnung, wobei insbesondere die Positionierung der wichtigsten Elemente erkennbar ist. Der Resonanzkörper 01 ist bei der hier dargestellten Ausführungsform als zylindrischer Hohlkörper gebildet, der aus dem oben genannten Gemisch aus Quarzsand und Polymer besteht. Die Wandungsstärke des Resonanzkörpers beträgt 0,5 bis 2,5 cm, bei einer axialen Länge von 20 bis 30 cm und einem Durchmesser von 10 bis 20 cm. Der Resonanzkörper 01 ist durch Trägerplatten 21 an seinen beiden Stirnseiten verschlossen. Die Trägerplatten 21 können aus Kunststoff bestehen und dienen gleichzeitig der Befestigung weiterer Bestandteile der Generatoranordnung. In geringem Abstand von jeder Trägerplatte 21 sind die beiden bifilar gewickelten Spulen 05 angebracht, so dass sie sich innerhalb des Resonanzkörpers 01 befinden. Außerdem ist an den Trägerplatten jeweils eine Antennenzuleitung 22 angebracht, welche die beiden Dipolantenennen 07 versorgen. An der Innenwandung des Resonanzkörpers 01 können die beiden Generatorschaltungen 03 befestigt werden, welche die nachgeordneten Spulen und Antennen versorgen. Außerdem verbleibt im Inneren des Resonanzkörpers genügend Raum zur Anordnung weitere Schaltungseinheiten oder zur Leitungsführung (nicht dargestellt).

Wie aus der Fig. 2 ersichtlich ist, füllen die beiden Antennen 07 im Wesentlichen den Durchmesser des Resonanzkörpers 01 aus, ohne dabei an der Wandung anzuliegen. Es ist zweckmäßig, wenn die beiden Dipolantennen im Wesentlichen als kreisförmige Faltdipole ausgebildet sind und sich bezogen auf die Achsrichtung des Resonanzkörpers 01 in einem Überdeckungsabschnitt überschneiden, ohne dort elektrisch kontaktiert zu sein. Der Überdeckungsabschnitt kann vorzugsweise 1/16 bis 1/4 der von der Dipolantenne umfassten Fläche ausmachen. Jeweils eine Antennenzuleitung 23 erstreckt sich ausgehend von der jeweiligen Trägerplatte 21 in etwa über die halbe Länge des Resonanzkörpers 01 und verläuft damit in einer parallelen Ebene zu der jeweils zweiten Antenne 07. Da die beiden Antennen 07 flächig ausgebildet sind, können sie innerhalb des Resonanzkörpers 01 beabstandet voneinander angeordnet werden, sodass eine elektrische Kontaktierung vermieden wird. Auf zusätzliche Isolationsmaßnahmen kann daher verzichtet werden, sodass die Abstrahlungsleistung der Antenne nicht beeinträchtigt wird.

Die weiteren elektrischen und elektronischen Komponenten der Generatorschaltung, wie sie in Fig. 1 dargestellt wurden, können innerhalb des Resonanzkörpers angeordnet sein oder vorzugsweise in einem eigenständigen Gehäuse 25, welches mit einer der beiden Trägerplatten 21 verbunden ist. Schließlich ist der Resonanzkörper 01 an seiner Außenseite mit der Messspule 13 umwickelt, wobei das in dieser Messspule induzierte Signal ein Maß für die auftretenden Resonanzschwingungen innerhalb des Resonanzkörpers darstellt. Aufgrund der im Resonanzkörper 01 angeordneten aktiven Bauelemente entsteht im Inneren des Resonanzkörpers ein so genanntes Wellenchaos, welches die gewünschten Resonanzwellen hervorruft.

Zum Schutz des Resonanzkörpers 01 und zur optischen Gestaltung kann an dessen Außenseite eine Schutzfolie 27 angebracht sein.

### Bezugszeichenliste

- 01: Resonanzkörper
- 03: Anregungsgenerator
- 05: Spule
- 07: Antenne
- 09: Oszillatorschaltkreis
- 11: -
- 13: Messspule
- 15: Graetzbrücke
- 17: Digitales Display
- 18: Betriebsanzeige
- 19: Kleinakkumulator
- 21: Trägerplatten
- 23: Antennenzuleitung
- 25: Gehäuse
- 27: Schutzfolie

## Patentansprüche

1. Generatoranordnung zur Erzeugung von Energiewellen, umfassend eine Oszillatorschaltung mit einem ersten und einem zweiten Anregungsgenerator (03), welche jeweils hochfrequente Anregungswellen über je eine vorgeschaltete Spule (05) an je eine Antenne (07) abgeben **dadurch gekennzeichnet, dass** die beiden Antennen (07) elektromagnetisch an einen Resonanzkörper (01) gekoppelt sind, in welchem sich in Reaktion auf die eingeprägten Anregungswellen Resonanzwellen ausbilden.

2. Generatoranordnung nach Anspruch 1, **dadurch gekennzeichnet, dass** die Anregungswellen eine Frequenz von 10 bis 15 MHz, insbesondere 12 MHz besitzen.

3. Generatoranordnung nach Anspruch 1 oder 2, **dadurch gekennzeichnet, dass** die den Antennen (07) vorgeschalteten Spulen als bifilar gewickelte Spulen (05) ausgebildet sind.

4. Generatoranrodung nach Anspruch 3, **dadurch gekennzeichnet, dass** die bifilar gewickelten Spulen (05) mit jeweils 5 bis 15 Windungen, insbesondere 9 Windungen gewickelt sind.

5. Generatoranordnung nach einem der Ansprüche 1 bis 4, **dadurch gekennzeichnet, dass** der Resonanzkörper (01) aus einem gesinterten Materialgemisch aus Kunststoff und Quarzsand besteht.

6. Generatoranordnung nach Anspruch 5, **dadurch gekennzeichnet, dass** der Resonanzkörper (01) aus einem Material besteht, welches gemäß dem Europäischen Patent EP 1 299 321 hergestellt wurde.

7. Generatoranordnung nach einem der Ansprüche 1 bis 6, **dadurch gekennzeichnet, dass** die Antennen (07) als Dipol ausgebildet sind.

8. Generatoranordnung nach Anspruch 7, **dadurch gekennzeichnet, dass** es sich bei den von den Dipolen (07) abgestrahlten Anregungswellen um transversale und longitudinale Wellen handelt.

9. Generatoranordnung nach einem der Ansprüche 1 bis 8, **dadurch gekennzeichnet, dass** der Resonanzkörper (01) als Hohlzylinder ausgebildet ist, in welchem die Spulen (05) und die Antennen (07) angeordnet sind.

10. Generatoranordnung nach Anspruch 9, **dadurch gekennzeichnet, dass** die Antennen (07) als kreisförmige Faltdipolantennen ausgebildet sind.

11. Generatoranordnung nach Anspruch 10, **dadurch gekennzeichnet, dass** die beiden Faltdipolantennen (07) in parallelen Ebenen innerhalb des Resonanzkörpers (01) angeordnet sind und sich in einer parallel zur Längsachse des Resonanzkörpers (01) liegenden Projektionsebene teilweise überdecken.

12. Generatorschaltung nach einem der Ansprüche 1 bis 11, **dadurch gekennzeichnet, dass** am Resonanzkörper (01) eine Messspule (13) angebracht ist, wobei die darin induzierte Spannung ein Maß für die vom Resonanzkörper abgegebenen Resonanzwellen ist.

13. Generatoranordnung nach Anspruch 12, **dadurch gekennzeichnet, dass** die von der Messspule (13) abgegebene Spannung über eine Graetzbrücke (15) gleichgerichtet wird.

14. Generatoranordnung nach Anspruch 11 oder 12, **dadurch gekennzeichnet, dass** die Messspule (13) außen um den Resonanzkörper (01) gewickelt ist.
